# EUROPEAN PATENT SPECIFICATION

(11) **EP 4 106 757 B1**
(45) Date of publication and mention of the grant of the patent: **23.08.2023**
(21) Application number: 21714345.2
(22) Date of filing: 16.03.2021
(51) Int. Cl.: A61K 31/444, A61K 45/06, A61P 11/00, A61P 29/00

(54) **METHODS INVOLVING NEUTROPHIL ELASTASE INHIBITOR ALVELESTAT FOR TREATING RESPIRATORY DISEASE MEDIATED BY ALPHA-1 ANTITRYPSIN DEFICIENCY**
METHODEN BEINHALTEND NEUTROPHILE ELASTASE INHIBITOR ALVELESTAT ZUR BEHANDLUNG VON ATEMWEGSERKRANKUNG AUFGRUND VON ALPHA-1 ANTITRYPSIN MANGEL
PROCÉDÉS IMPLIQUANT L'ALVÉLESTAT INHIBITEUR DE L'ÉLASTASE NEUTROPHILE POUR LE TRAITEMENT D'UNE MALADIE RESPIRATOIRE INDUITE PAR UNE DÉFICIENCE EN ALPHA-1 ANTITRYPSINE

(30) Priority: 16.04.2020 GB 202005519; 16.04.2020 GB 202005520; 04.08.2020 US 202062706195 P
(43) Date of publication of application: 28.12.2022
(73) Proprietor: Mereo Biopharma 4 Limited, London Greater London W1G 0QF (GB)
(72) Inventor: PARKIN, Jacqueline, London W1G 0QF (GB)
(74) Representative: Carpmaels & Ransford LLP
(86) International application number: PCT/GB2021/050653
(87) International publication number: WO 2021/209739

(56) References cited:
- WO-A1-2010/094964
- WO-A1-2016/016364
- Anonymous: "Alvelestat (MPH966) for the Treatment of ALpha-1 ANTitrypsin Deficiency", , 21 September 2018 (2018-09-21), XP055810968, Retrieved from the Internet: URL:https://clinicaltrials.gov/ct2/show/NC T03679598 [retrieved on 2021-06-07]
- VON NUSSBAUM FRANZ ET AL: "Neutrophil elastase inhibitors for the treatment of (cardio)pulmonary diseases: Into clinical testing with pre-adaptive pharmacophores", BIORGANIC & MEDICINAL CHEMISTRY LETTERS, ELSEVIER, AMSTERDAM , NL, vol. 25, no. 20, 20 August 2015 (2015-08-20), pages 4370-4381, XP029285851, ISSN: 0960-894X, DOI: 10.1016/J.BMCL.2015.08.049

## Description

### FIELD OF THE INVENTION

The present invention, which is defined by the claims, relates to 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1 ,2-dihydropyridine-3-carboxamide and salts thereof for use in a method of treating a disease of the respiratory system which is mediated by α-1 antitrypsin deficiency, wherein the disease is selected from chronic obstructive pulmonary disease (COPD), emphysema, asthma and bronchiectasis, in a patient in need thereof who has not responded to previous alpha-1 antitrypsin (AAT) therapy.

### BACKGROUND OF THE INVENTION

Elastases are possibly the most destructive enzymes in the body, having the ability to degrade virtually all connective tissue components.

Human neutrophil elastase (hNE), a member of the chymotrypsin superfamily of serine proteases is a 33-KDa enzyme stored in the azurophilic granules of the neutrophils. In neutrophils the concentration of NE (neutrophil elastase) exceeds 5 mM and its total cellular amount has been estimated to be up to 3 pg. Upon activation, NE is rapidly released from the granules into the extracellular space (See Kawabat et al. 2002, Eur. J. Pharmacol. 451,1-10). The main intracellular physiological function of NE is degradation of foreign organic molecules phagocytosed by neutrophils, whereas the main target for extracellular elastase is elastin (Janoff and Scherer, 1968, J. Exp. Med. 128, 1137-1155). NE is unique, as compared to other proteases (for example, proteinase 3) in that it has the ability to degrade almost all extracellular matrix and key plasma proteins (See Kawabat et al. , 2002, Eur. J. Pharmacol. 451,1-10), including elastin, Type 3 and type 4 collagens, laminin, fibronectin, cytokines, etc. (Ohbayashi, H., 2002, Expert Opin. Investig. Drugs, 11, 965-980). NE is a major common mediator of many pathological changes seen in chronic lung disease (Stockley, R. A. 1994, Am. J. Resp. Crit. Care Med. 150,109-113).

The destructive role of NE was solidified almost 40 years ago when Laurell and Eriksson reported an association of chronic airflow obstruction and emphysema with deficiency of serum α1-antitrypsin (Laurell and Eriksson, 1963, Scand. J. Clin. Invest. 15,132-140), which was found to be the most important endogenous inhibitor of NE. The imbalance between NE and endogenous antiprotease is believed to cause excess NE in pulmonary tissues which is considered as a major pathogenic factor in chronic obstructive pulmonary disease (COPD). The excessive NE destroys normal pulmonary structures, followed by the irreversible enlargement of the respiratory airspaces, as seen mainly in emphysema. There is an increase in neutrophil recruitment into the lungs which is associated with increased lung elastase burden and emphysema in α1-proteinase inhibitor-deficient mice (Cavarra et al., 1996, Lab. Invest. 75, 273-280). Individuals with higher levels of the NE- α1 protease inhibitor complex in bronchoalveolar lavage fluid show significantly accelerated decline in lung functions compared to those with lower levels (Betsuyaku et al. 2000, Respiration, 67,261-267). Instillation of NE via the trachea in rats causes lung haemorrhage, neutrophil accumulation during acute phase and emphysematous changes during chronic phase (Karaki et al., 2002, Am. J. Resp. Crit. Care Med., 166, 496-500). Studies have shown that the acute phase of pulmonary emphysema and pulmonary haemorrhage caused by NE in hamsters can be inhibited by pre-treatment with inhibitors of NE (Fujie et al., 1999, Inflamm. Res. 48,160-167).

Acute lung injury caused by endotoxin in experimental animals is associated with elevated levels of NE (Kawabata, et al., 1999, Am. J. Resp. Crit. Care, 161, 2013-2018). Acute lung inflammation caused by intratracheal injection of lipopolysaccharide in mice has been shown to elevate the NE activity in bronchoalveolar lavage fluid which is significantly inhibited by a NE inhibitor (Fujie et al. , 1999, Eur. J. Pharmacol., 374,117-125 ; Yasui, et al. , 1995, Eur. Resp. J. , 8,1293-1299). NE also plays an important role in the neutrophil- induced increase of pulmonary microvascular permeability observed in a model of acute lung injury caused by tumour necrosis factor < x (TNFce) and phorbol myristate acetate (PMA) in isolated perfused rabbit lungs (Miyazaki et al. , 1998, Am. J. Respir. Crit. Care Med., 157, 89-94).

NE has been implicated in the promotion or exacerbation of a number of diseases such as pulmonary emphysema, pulmonary fibrosis, adult respiratory distress syndrome (ARDS), ischemia reperfusion injury, rheumatoid arthritis and pulmonary hypertension.

The present invention discloses dosage forms and dosage regimens of 2-pyridione derivatives that are inhibitors of NE and homologous serine proteases such as proteinase 3 and pancreatic elastase, and are thereby useful in therapy.

WO 2005/026123 teaches a class of NE inhibitors that are useful in therapy. WO 2005/026123 further discloses a specific NE inhibitor compound identified therein as 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide and salts thereof (Example 94, page 85). This compound is designated herein as compound (I). Compound (I) is also known as alvelestat, AZD9668, or MPH966.

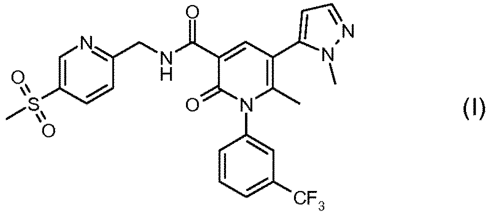

Similarly, WO 2010/094964 teaches salt forms of compound (I), as shown above with respect to WO 2005/026123. This document reports that compound (I) is administered in a maximum daily amount of 200 mg per day.

Compound (I) may be useful in the treatment of diseases of the respiratory tract/system such as obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; adult respiratory distress syndrome (ARDS); lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, pulmonary hypertension, and infection due to respiratory syncytial virus, influenza, coronavirus (including SARS) and adenovirus.

There is a significant need for avoiding the initial activation of NE. Once activation has taken place, the inflammatory cascade that follows can cause irreversible damage, and itself is difficult to reverse. Therefore, the present invention aims to provide highly effective NE inhibition, by dose regimens of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide and salts thereof, that achieve and sustain blood levels at or above those needed to inhibit NE throughout the dosing period and in particular prevent the initial activation of NE.

WO 2016/016364 relates to substituted bicyclic dihydropyrimidinones and their use as inhibitors of neutrophil elastase activity, pharmaceutical compositions containing the same, and methods of using the same as agents for treatment and/or prevention of pulmonary, gastrointestinal and genitourinary diseases, inflammatory diseases of the skin and the eye and other autoimmune and allergic disorders, allograft rejection, and oncological diseases.

"Alvelestat (MPH966) for the Treatment of ALpha-1 ANTitrypsin Deficiency", 21st September 2018, ClinicalTrials.gov archive, XP055810968, describes a study to evaluate alvelestat for its safety and tolerability as well as the mechanistic effect of oral administration of alvelestat in subjects with confirmed AATD defined as Pi*ZZ, Pi*SZ, Pi*null, or another rare phenotype/genotype known to be associated with either low (serum AAT level <11 µM or <57.2 mg/dL) or functionally impaired AAT including "F" or "I" mutations.

Nussbaum et al (Neutrophil elastase inhibitors for the treatment of (cardio)pulmonary diseases: Into clinical testing with pre-adaptive pharmacophores, Bioorganic & Medicinal Chemistry Letters, Volume 25, Issue 20,2015, Pages 4370-4381, ISSN 0960-894X, https://doi.org/10.1016/j.bmcl.2015.08.049) reviews the development of effective human neutrophil elastase inhibitors, focusing on recent developments.

WO 2010/094964 relates to 6-Methyl-5-(I-methyl-1H-pyrazol-5-yl)-N-{ [5-(methylsulfonyl)pyridin-2-yl]methyl }-2-oxo-1-[3-(trifluoromethyl)phenyl]-1,2-dihydropyridine-3-carboxamide 4-methylbenzenesulfonate and a crystalline form thereof, together with processes for preparing such salt and form, pharmaceutical compositions comprising such a form, and the use of such a salt and form in therapy.

### SUMMARY OF THE INVENTION

The present invention is defined by the claims. In an aspect the invention provides alvelestat or a salt thereof for use in a method of treating a disease of the respiratory system which is mediated by α-1 antitrypsin deficiency, wherein the disease is selected from chronic obstructive pulmonary disease (COPD), emphysema, asthma and bronchiectasis, in a patient in need thereof who has not responded to previous alpha-1 antitrypsin (AAT) therapy, the method comprising administering an effective amount of alvelestat or a salt thereof to the patient, and wherein the amount of alvelestat or a salt thereof administered is 120-300 mg BID.

In the invention a solid dosage form may be used, comprising 120-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof.

In the invention a solid dosage form may be used comprising 220-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof.

In the invention a single or multiple solid dosage form(s) may be used for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of a disease of the respiratory system which is mediated by α-1 antitrypsin deficiency, said single or multiple solid dosage form(s) comprising a total of 120-300 mg of 6-methyl-5-(I-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration.

In the invention a single or multiple solid dosage form(s) may be used for oral administration of 6-methyl-5-(I-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of a disease of the respiratory system which is mediated by α-1 antitrypsin deficiency, said single or multiple solid dosage form(s) comprising a total of 220-300 mg of 6-methyl-5-(I-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration.

In the invention a single or multiple solid dosage form(s) may be used for oral administration of 6-methyl-5-(I-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of a disease of the respiratory system which is mediated by α-1 antitrypsin deficiency, said single or multiple solid dosage form(s) comprising a total of 120-300 mg of 6-methyl-5-(I-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations.

In the invention a single or multiple solid dosage form(s) may be used for oral administration of 6-methyl-5-(I-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of a disease of the respiratory system which is mediated by α-1 antitrypsin deficiency, said single or multiple solid dosage form(s) comprising a total of 220-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations.

In the invention a kit may be used, comprising a plurality of solid dosage forms for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequentially administering a total of 120-300 mg of said 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations, for the treatment of a disease of the respiratory system which is mediated by α-1 antitrypsin deficiency.

In the invention a kit may be used, comprising a plurality of solid dosage forms for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequentially administering a total of 220-300 mg of said 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations, for the treatment of a disease of the respiratory system which is mediated by α-1 antitrypsin deficiency.

In the invention a kit may be used, comprising a plurality of solid dosage forms for oral administration, each dosage form comprising 120-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequential, twice daily administration of each dose, for the treatment of a disease of the respiratory system which is mediated by α-1 antitrypsin deficiency.

In the invention a kit may be used, comprising a plurality of solid dosage forms for oral administration, each dosage form comprising 220-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequential, twice daily administration of each dose, for the treatment of a disease of the respiratory system which is mediated by α-1 antitrypsin deficiency.

The invention may be used for treating a disease of the respiratory system which is mediated by α-1 antitrypsin deficiency, comprising the twice daily oral administration of a single or multiple solid dosage form(s), said single or multiple solid dosage form(s) comprising a total of 120-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, to a human patient suffering from a disease of the respiratory system which is mediated by α-1 antitrypsin deficiency.

The invention may be used for treating a disease of the respiratory system which is mediated by α-1 antitrypsin deficiency, comprising the twice daily oral administration of a single or multiple solid dosage form(s), said single or multiple solid dosage form(s) comprising a total of 220-300 mg of 6-methyl-5-(1-methyl-1H-pyrazol-5-yl)-N-{[5-(methylsulfonyl)pyridin-2-yl]methyl}-2-oxo-1 -[3-(trifluoromethyl)phenyl]-1,2-dihydropyridine-3-carboxamide or a salt thereof per administration, to a human patient suffering from a disease of the respiratory system which is mediated by α-1 antitrypsin deficiency.

In the invention a single or multiple solid dosage form(s) may be used for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of COPD (Chronic Obstructive Pulmonary Disease), said single or multiple solid dosage form(s) comprising a total of 120-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration.

In the invention a single or multiple solid dosage form(s) may be used for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of COPD (Chronic Obstructive Pulmonary Disease), said single or multiple solid dosage form(s) comprising a total of 220-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration.

In the invention a single or multiple solid dosage form(s) may be used for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of COPD (Chronic Obstructive Pulmonary Disease), said single or multiple solid dosage form(s) comprising a total of 120-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations.

In the invention a single or multiple solid dosage form(s) may be used for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for the treatment of COPD (Chronic Obstructive Pulmonary Disease), said single or multiple solid dosage form(s) comprising a total of 220-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations.

In the invention a kit may be used, comprising a plurality of solid dosage forms for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequentially administering a total of 120-300 mg of said 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations, for the treatment of COPD (Chronic Obstructive Pulmonary Disease).

In the invention a kit may be used, comprising a plurality of solid dosage forms for oral administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequentially administering a total of 220-300 mg of said 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, and wherein the administration of each dose takes place twice daily, with at least 8 hours between administrations, for the treatment of COPD (Chronic Obstructive Pulmonary Disease).

In the invention a kit may be used, comprising a plurality of solid dosage forms for oral administration, each dosage form comprising 120-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequential, twice daily administration of each dose, for the treatment of COPD (Chronic Obstructive Pulmonary Disease).

In the invention a kit may be used, comprising a plurality of solid dosage forms for oral administration, each dosage form comprising 220-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, said kit containing instructions for sequential, twice daily administration of each dose, for the treatment of COPD (Chronic Obstructive Pulmonary Disease).

The invention may be used for treating COPD (Chronic Obstructive Pulmonary Disease), comprising the twice daily oral administration of a single or multiple solid dosage form(s), said single or multiple solid dosage form(s) comprising a total of 120-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, to a human patient suffering from COPD.

The invention may be used for treating COPD (Chronic Obstructive Pulmonary Disease), comprising the twice daily oral administration of a single or multiple solid dosage form(s), said single or multiple solid dosage form(s) comprising a total of 220-300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof per administration, to a human patient suffering from COPD.

Chronic Obstructive Pulmonary Disease (COPD) is an example of a disease of the respiratory system which is mediated by α-1 antitrypsin deficiency.

Regarding diseases of the respiratory system and NE, and in particular, COPD and NE, there is a clear pathological link as deficiency of α1-antitrypsin (AATD) is a specific risk factor for development of lung damage manifesting as COPD. The main physiological effect of α1-antitrypsin is to neutralise NE. It is considered that it is the unopposed NE activity that drives lung tissue destruction in AATD related COPD, particularly emphysema. The lung is particularly susceptible for two reasons: (1) the day to day inhalation of noxious substances, in particular cigarette smoke, are powerful activators of neutrophils causing release of NE; (2) Elastin - the target of elastase is a major component of lung tissue (more so than other tissues) and without protection of NE inhibitors the elastin that supports delicate structures of the lung is broken down and the lung tissue progressively destroyed.

Hence, in all of the aspects of the invention, the disease of the respiratory system, in particular COPD is preferably associated with α1-antitrypsin deficiency (AATD). Similarly, in all of the aspects of the invention, the disease of the respiratory system, for example COPD is preferably treated via NE inhibition.

### BRIEF DESCRIPTION OF THE FIGURES

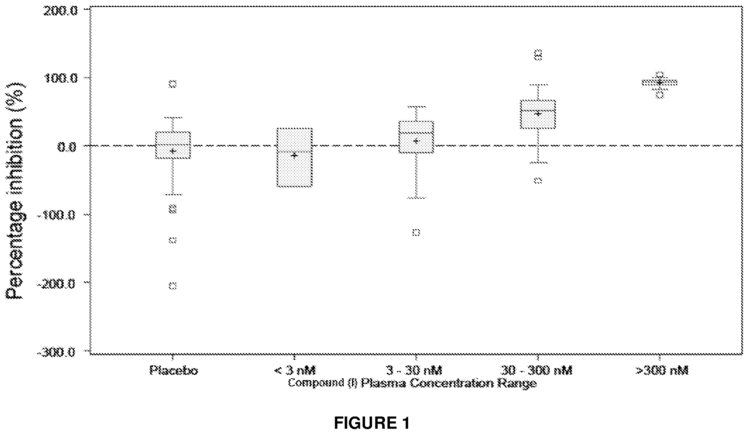

Figure 1 shows a box plot of percentage inhibition of zymosan-stimulated NE activity in plasma by plasma PK concentration (Multiple Ascending Dose study). Note: the dot is the mean value. The line inside the box represents the median (50th percentile). The upper and lower edges of the box represent the 75th and 25th percentiles respectively, i.e. the inter quartile range (IQR). The whiskers represent the extent of the data with 1.5*IQR beyond the upper and lower percentiles. Outliers from this range are shown.

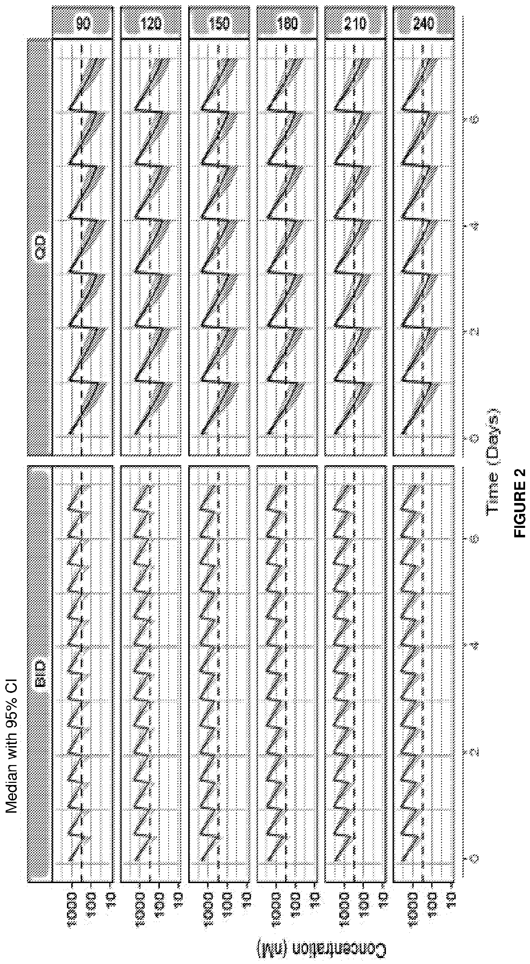

Figure 2 shows population PK simulated median plasma concentration absolute values for the tosylate salt of doses of up to 240 mg given BID (twice daily) or QD (once daily) 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide, showing the 95% confidence intervals (CI) for trough concentrations (Cₘᵢₙ).

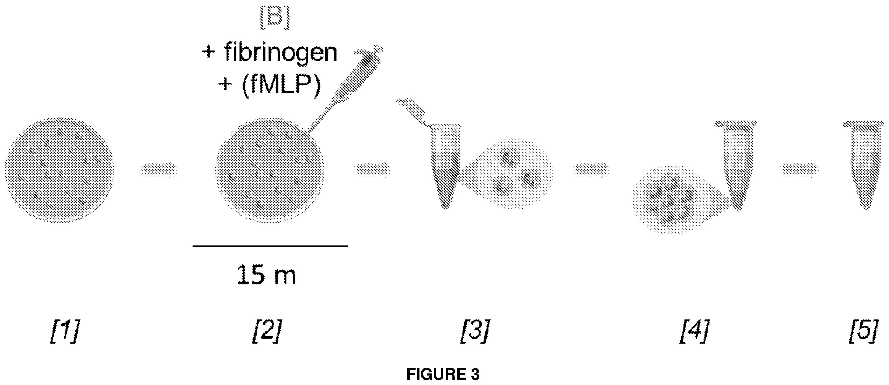

Figure 3 shows a visualisation of proteinase/anti-proteinase imbalance model methodology. The following steps are shown. [1] plating of isolated neutrophils at 1 million cells/ml; [2] addition of substrate (fibrinogen) and stimulus (fMLP) beginning a 15 minute incubation period; [3] removal of treated cells from plate and centrifugation; [4-5] transfer of supernatant and disposal of cells. [B] indicates point of alvelestat addition to plated neutrophils alongside the addition of substrate and stimuli, namely "one-step" incubation.

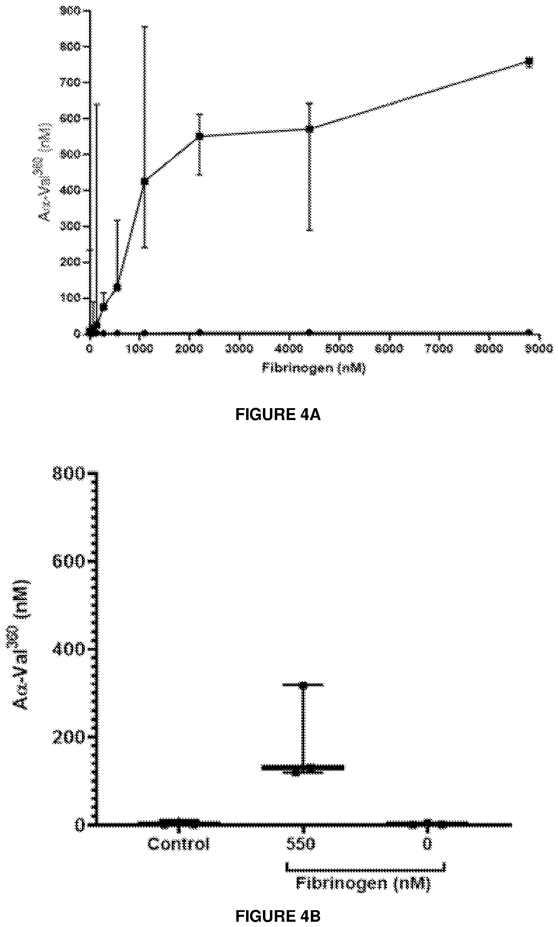

Figure 4 shows that increasing fibrinogen within a closed model increases elastase signal. **A** Aα-Val³⁶⁰ levels produced by 1 million neutrophils/ml when incubated with increasing concentrations of uncleaved fibrinogen over 60 minutes; ●= with neutrophils, ■ = without neutrophils; n=3; error bars are median +/- IQR; **B** Quantity of Aα-Val³⁶⁰ produced by 1 million neutrophils/ml when exposed to 550 nM uncleaved fibrinogen; n=3; normality determined by Shapiro-Wilk normality test; 0 to 550 significance determined by unpaired t-test; 550 to control significance determined by Mann-Whitney test; error bars mean +/- SEM.

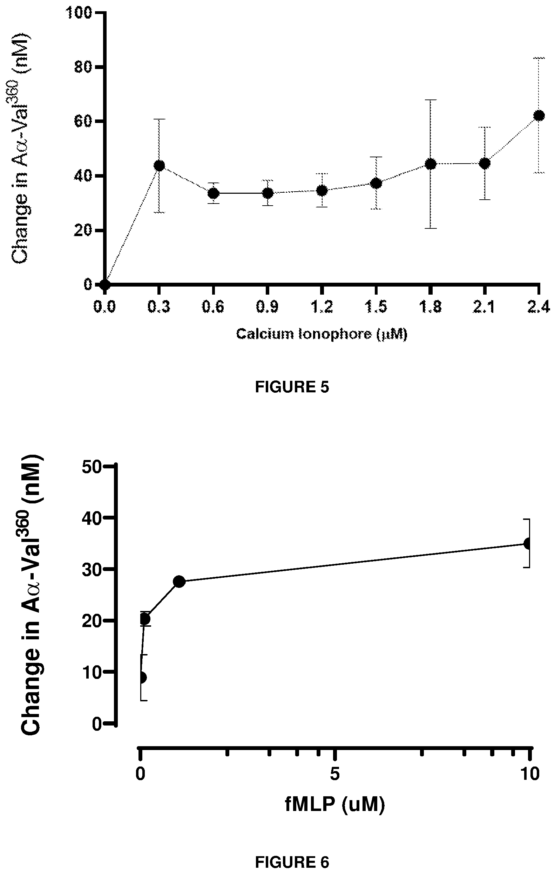

Figure 5 shows proteinase activity following stimulation of neutrophils with Cal. Baseline corrected Aα-Val³⁶⁰ levels following stimulation of neutrophils at 2.5 million/ml with Cal at 2.4/2.1/1.8/1.5/1.2/0.9/0.6/0.3 µM with fibrinogen (550 nM) for 60 minutes; n=3.
Figure 6 shows proteinase activity following stimulation of neutrophils with fMLP. Change in Aα-Val³⁶⁰ levels following stimulation of neutrophils at 2.5 million/ml with fMLP at 0, 0.1,1,10 µM fMLP with fibrinogen (550 nM) for 15 minutes; n=3.

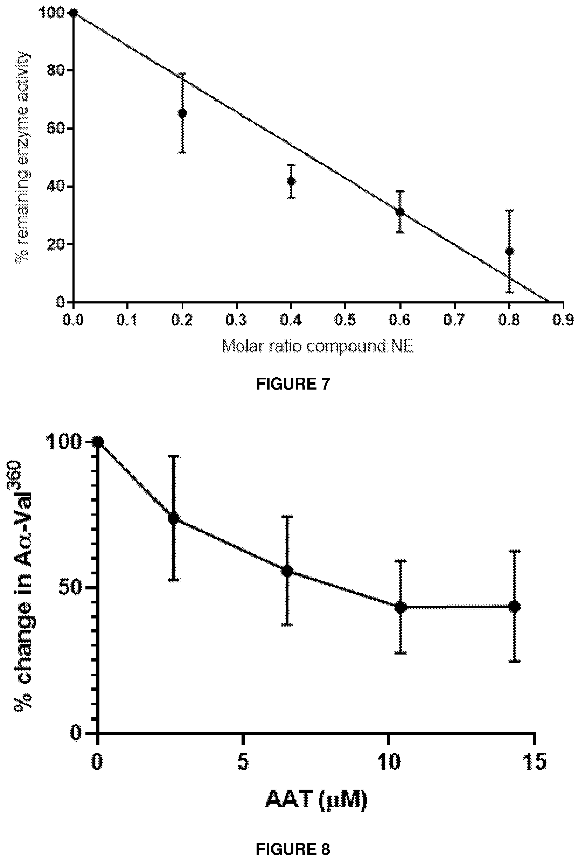

Figure 7 shows assessment of alvelestat activity. Activity of alvelestat when in the presence of increasing concentrations of NE; n=4; error bars mean +/- SEM; line of best fit calculated using simple linear regression and with control fixed at 100%; normality determined using Shapiro-Wilk test.
Figure 8 shows that introduction of AAT into the imbalance model reduces NSP activity footprint, and the effect of increasing concentrations of active AAT on the activity neutrophil elastase activity within a model of proteinase/anti-proteinase imbalance. Percentage neutrophil elastase activity up to 15 µM with 0 µM AAT set as 100% activity baseline; n=4; mean +/- SEM.

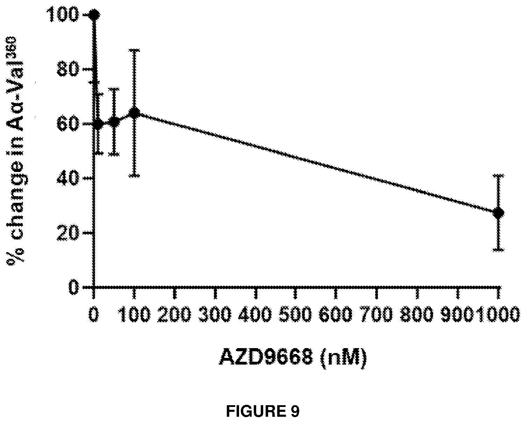

Figure 9 shows the inhibitory effect of AZD9668 (alvelestat) on proteinase activity. Percentage change in activity produced by increasing concentrations of alvelestat (at 10 nM, 50 nM, 100 nM, 1000 nM) on the activity of neutrophil elastase within a model of proteinase/anti-proteinase imbalance. No inhibitor as 100% activity baseline. Error bars mean +/- SEM.

### DETAILED DESCRIPTION OF THE INVENTION

It has been determined that, to avoid NE activation, hence, avoid damage associated with NE in diseases of the respiratory system which are mediated by α-1 antitrypsin deficiency, such as COPD, inhibitory treatment must achieve and maintain a threshold Cmin (trough plasma concentration) in a human of 300 nM or above. This is shown in Figure 1. Achieving and maintaining such a threshold Cmin is correlated with an increased chance of survival and better quality of life in such patients.

This requires a substantially higher dose of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide than was previously thought. For example, the prior art discloses that a maximum daily amount of 200 mg should be administered.

As used herein, a disease of the respiratory system which is mediated by α-1 antitrypsin deficiency includes asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; adult respiratory distress syndrome (ARDS); lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, pulmonary hypertension, and infection due to respiratory syncytial virus, influenza, coronavirus (including SARS) and adenovirus.

In a preferred embodiment, the disease of the respiratory system which is mediated by α-1 antitrypsin deficiency is chronic obstructive pulmonary disease (COPD). In another preferred embodiment, the disease of the respiratory system which is mediated by α-1 antitrypsin deficiency is emphysema. In another preferred embodiment, the disease of the respiratory system which is mediated by α-1 antitrypsin deficiency is bronchiectasis. In another preferred embodiment, the disease of the respiratory system which is mediated by α-1 antitrypsin deficiency is asthma.

As used herein, a disease of the respiratory system which is mediated by α-1 antitrypsin deficiency includes a patient with α-1 antitrypsin deficiency who is suffering from a disease of the respiratory system described herein, e.g. chronic obstructive pulmonary disease (COPD) or emphysema.

As used herein, a disease which is mediated by α-1 antitrypsin deficiency includes a patient with α-1 antitrypsin deficiency who is suffering from a disease. As used herein, a disease which is mediated by α-1 antitrypsin deficiency includes panniculitis.

Threshold Cₘᵢₙ values have been shown to be achieved and maintained when a patient suffering from a disease of the respiratory system which is mediated by α-1 antitrypsin deficiency is administered a 120 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, twice daily. Preferably, a minimum of 120 mg, twice per day is administered to the human patient suffering from a disease of the respiratory system which is mediated by α-1 antitrypsin deficiency, for example, COPD. Thus, a minimum total daily dose of 240mg, preferably 440 mg, more preferably 480 mg is administered.

In further embodiments of the invention, a minimum of 180 mg of alvelestat or a salt thereof, twice per day is administered to the human patient suffering from a disease of the respiratory system which is mediated by α-1 antitrypsin deficiency, for example, COPD. Thus, a minimum total daily dose of 360 mg, preferably 540 mg, is administered.

Furthermore, such threshold Cₘᵢₙ values have been shown to be achieved and maintained when a patient suffering from a respiratory disease mediated by α-1 antitrypsin deficiency, for example COPD, is administered a 240 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, twice daily. Preferably, a minimum of 240 mg, twice per day is administered to the human patient suffering from a disease of the respiratory system which is mediated by α-1 antitrypsin deficiency, for example COPD. Thus, a minimum total daily dose of 440 mg, preferably 480 mg is administered.

Cₘᵢₙ is a term used in pharmacokinetics for the minimum blood plasma concentration reached by a drug prior to administration of a second dose. E.g. Cₘᵢₙ can be the trough plasma concentration observed in Figure 2. Cₘᵢₙ is the opposite of Cₘₐₓ, the maximum concentration that the drug reaches (e.g. peak plasma concentration).

For all of the aspects of the invention disclosed herein, the administered alvelestat or a salt thereof results in a Cₘᵢₙ plasma concentration of at least 300 nM in the patient.

For all of the aspects of the invention disclosed herein, preferably the administered alvelestat or a salt thereof results in a Cₘₐₓ plasma concentration of at least 1000 nM in the patient.

For all of the aspects of the invention disclosed herein, preferably the administered alvelestat or a salt thereof results in a Cₘᵢₙ plasma concentration of at least 300 nM and a Cₘₐₓ plasma concentration of at least 1000 nM in the patient.

For all of the aspects of the invention disclosed herein, each dosage form is preferably a single solid entity. However, each administration may comprise a plurality of solid dosage forms, subject to each administration having no more than 300 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, and preferably 240-480 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof.

Twice daily administration means that the administration takes place at regular spaced intervals, preferably at least 8 hours apart, more preferably at least 10 hours apart, for example about 12 hours apart ± 1 hour.

For all of the aspects of the invention disclosed herein, preferably a maximum daily dose of 600 mg is not exceeded, more preferably not more than 500 mg.

For all of the aspects of the invention disclosed herein, preferably each individual dose contains between 120 and 250 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, for example 120 mg, 180 mg or 240 mg.

For all of the aspects of the invention disclosed herein, preferably each individual dose contains between 160 and 250 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, e.g. 180 mg.

For all of the aspects of the invention disclosed herein, preferably each individual dose contains between 220 and 250 mg of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof, e.g. 240 mg.

The administration of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof preferably takes place chronically. Hence, a preferred embodiment of a kit comprises a defined supply of dosage forms, such as a weekly, bi-weekly or monthly supply of dosage forms of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof.

In an embodiment of the invention, the salt of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide is the tosylate salt of 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide.

6-Methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide, or a salt thereof, may be administered to an individual in accordance with an effective dosing regimen for a desired period of time or duration, such as at least one week, at least about one month, at least about 2 months, at least about 3 months, at least about 6 months, at least about 12 months, at least about 24 months, or longer. For example, the compound may be administered on a daily or intermittent schedule for the duration of the subject's life.

The dose of 6-Methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide or a salt thereof may be administered according to a dosage escalation regime of the invention. This allows safe titration up to a daily dose of alvelestat, e.g. of 120mg twice daily (BID). For example, a dosage escalation regime up to a 120mg twice daily dose of alvelestat according to the invention comprises administration of alvelestat or a salt thereof at a dose of 60mg of alvelestat twice daily for a first period of time, followed by 120mg twice daily thereafter. The first period may be from 5-20 days, preferably each about one week (7 days). In particular, alvelestat or a salt thereof is administered at 60mg twice daily for one week, followed by 120mg twice daily thereafter. Doses are referred to as the equivalent amount of alvelestat free base.

A dose escalation regime may also be used to allow safe titration up to a daily dose of alvelestat, e.g. of 240mg twice daily (BID). For example, a dosage escalation regime up to a 240mg twice daily dose of alvelestat according to the invention comprises administration of alvelestat or a salt thereof at a dose of 60mg of alvelestat twice daily for a first period of time, followed by 120mg twice daily for a second period of time, followed by 180mg twice daily for a third period of time, and 240mg twice daily thereafter. The first, second and third periods may each be from 5-20 days, preferably each about one week (7 days). In particular, alvelestat or a salt thereof is administered at 60mg twice daily for one week, followed by 120mg twice daily for one week, followed by 180mg twice daily for one week, and 240mg twice daily thereafter. Doses are referred to as the equivalent amount of alvelestat free base.

In an embodiment of the invention, the amount (e.g. mg) of alvelestat or a salt thereof referred to in the amount administered, the dose or the solid dosage form, is the corresponding amount of alvelestat free base in the alvelestat or salt thereof. For example, if a method of the invention requires the administration of a dose of 240mg of alvelestat or a salt thereof, the amount of an alvelestat salt used will be equivalent to 240 mg of alvelestat freebase.

As shown in the examples, alvelestat Cmax concentrations achieved by the described dosing regimen suppressed neutrophil elastase (NE) activity by 72.6% (see Figure 9). By contrast, AAT at concentrations achieved therapeutically in treatment of alpha-1 deficiency, by use of AAT replacement "augmentation", suppressed NE activity by only 56.7% (see Figure 8). Thus, alvelestat may be a more potent inhibitor of NE than AAT augmentation therapy. It is therefore believed that alvelestat may be useful in treating patients who have not responded well to AAT augmentation therapy. Such augmentation therapy involves the administration of AAT intravenously in order to slow down progression of diseases of the respiratory system which are mediated by AATD.

Thus, the invention provides alvelestat or a salt thereof for use in a method of treating a disease of the respiratory system which is mediated by α-1 antitrypsin deficiency (e.g. COPD) in a patient in need thereof who has not responded to AAT therapy, comprising administering an effective amount of alvelestat or a salt thereof as disclosed herein (e.g. 120-300 mg BID, in particular 220-300mg BID) to the patient. In an embodiment, the administration of alvelestat or a salt thereof is either in place of, or in combination with, AAT augmentation therapy. Non-responders may be patients for whom AAT does not treat or delay progression of a disease of the respiratory system which is mediated by AATD, e.g. COPD.

Therefore, for all of the aspects of the invention disclosed herein, the patient in need of treatment may have previously been treated for α-1 antitrypsin deficiency, or is currently treated, by the administration of AAT. The compound (I) or salt thereof may be used alone when appropriate, or in the form of appropriate pharmaceutical composition comprising the compound of the invention in combination with a pharmaceutically acceptable diluent, adjuvant or carrier. Particularly preferred are compositions not containing material capable of causing an adverse reaction, for example, an allergic reaction.

The solid dosage form or forms as defined herein are preferably in the form of a tablet, tablets, capsule or capsules, most preferably a tablet.

According to the invention, there is provided a pharmaceutical composition comprising a compound (I) or salt thereof in admixture with a pharmaceutically acceptable diluent or carrier. The compound (I) or salt thereof is preferably used in a micronised or a milled form. The compound (I) or salt thereof is preferably less than 50% by weight and more preferably less than 30% by weight of the total composition weight in the compositions described herein (including the oral compositions).

In an embodiment of the methods described herein, alvelestat or a salt thereof is administered to a human patient in need thereof.

For oral administration the compound (I) or salts thereof may, for example, be admixed with an adjuvant, diluent or a filler, for example, lactose, saccharose, sorbitol, mannitol, dibasic calcium phosphate (dicalcium phosphate) including hydrated and anhydrous forms; a starch, for example, potato starch, corn (maize) starch or amylopectin; a cellulose derivative such as microcrystalline cellulose (MCC) or silicified microcrystalline cellulose (SMCC) and the like. In some embodiments mixtures of these may be used. In one embodiment the compound (I) tosylate is not admixed with mannitol. In one embodiment, the quantity of hydrophilic celluloses, such as MCC in the complete formulation ranges between 50 % and 98 %. These adjuvents, diluents and fillers are used in total in 60 to 98 parts, preferably in 70 to 95 parts, thereof per 100 parts of the solid formulation by weight. Examples of cellulose derivatives such as microcrystalline cellulose, include Avicel PHIOI, PH 102, PH 102 SCG, PH200, PH301, PH302, and PH-F20, Avicel RC-A591NF. An example of silicified microcrystalline cellulose products is ProSolv 90 HD, a mixture of MCC and colloidal silicon dioxide (manufactured by JRS Pharma). Examples of dibasic calcium phosphate dihydrate products are Calipharm D (from ThermoPhos), ICL D (from ICL Performance Products), Calstar (FMC Biopolymer), Di-Cafos (Chemische Fabrik Budenheim), DI-TAB (Innophos) and Emcompress (JRS Pharma LP). Examples of dibasic calcium phosphate anhydrate are ICL A (from ICL Performance Products), Fuji Calin (from Fuji Chemicals), A-TAB (Innophos), Di-Cafos AN (Chemische Fabrik Budenheim) and Emcompress Anhydrous (JRS Pharma LP). Examples of mannitol products is Pearlitol DC 300 and Pearlitol SD200 (manufactured by Roquette). An example of a lactose product is Pharmatose DCL 15 (manufactured by DMV).

A binder may be optionally used, for example, hydroxypropyl cellulose (HPC), hydroxypropylmethyl cellulose (HPMC), polyvinylpyrrolidone (PVP) or gelatine and 0.5 to 10 parts, preferably 1 to 4 parts, thereof is used per 100 parts of the solid pharmaceutical formulation by weight. An example of hydroxypropyl cellulose includes HPC LF. Examples of hydroxypropylmethyl cellulose include PVP K30 and PVP K90.

Disintegrating agents include for example, carmellose calcium, carboxymethyl starch sodium, croscarmellose sodium, crospovidone (crosslinked polyvinylpyrrolidone) and the like, and 0.5 to 15 parts, preferably 2 to 10 parts, thereof is used per 100 parts of the solid pharmaceutical formulation by weight. Disintegrating agents are exemplified by Kollidon CL (manufactured by BASF).

Lubricants include magnesium stearate, calcium stearate, sucrose esters of fatty acids, sodium stearyl fumarate, stearic acid, polyethyleneglycol, wax, paraffin and the like. The amount of lubricant is between 0.05 % and 5 % and is preferably between 0.5 % to 3.5 %, wherein the % is by weight of the formulation.

Surfactants include sodium lauryl sulfate, polysorbate 80, hydrogenated oil, polyoxyethylene(160)polyoxypropylene(30)glycol, and the like. The amount of surfactant is less than 2 %, suitably less than 1.5%, for example less than 1.1 % wherein the % is by weight of the formulation.

### EXAMPLES

### Example 1

The effect of alvelestat on ex vivo zymosan-stimulated neutrophil elastase (NE) activity in whole blood was evaluated. Blood from healthy volunteers from a Multiple Ascending Dose (MAD) alvelestat study was collected at baseline (before alvelestat treatment) and on the different doses of alvelestat treatment or placebo. Citrated blood samples were incubated with zymosan, and the resulting NE activity was measured by a fluorogenic assay based on the generation of the fluorescent cleavage product, 7-amino-4-methylcoumarin. NE suppressive effect of alvelestat or placebo was expressed as the percentage inhibition of the pre-treatment (baseline) zymosan stimulated NE. Alvelestat concentrations were measured in plasma by protein precipitation followed by liquid chromatography and tandem mass spectrometry, measured at the same timepoints as NE activity. Plasma concentrations were grouped for analysis as <3nM; 3-30 nm; 30-300 nM and >300 nM.

Figure 1 shows the percentage inhibition of zymosan stimulated neutrophil elastase (NE) in plasma at increasing concentrations of alvelestat, and indicates that -100% NE inhibition can be achieved by a plasma concentration of alvelestat of 300 nM or above.

### Example 2

Figure 2 shows population PK simulated median plasma concentrations (dark line) for the tosylate salt of doses of up to 240 mg given BID (twice daily) or QD (once daily) 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide, showing the 95% CI. The median and 95% CI bounds (in grey) show the predicted PK levels of 240 mg BID meet the greater than 300nM threshold (dotted line). Figure 2 also shows the predicted PK levels of 120 mg BID meet the 300nM threshold (dotted line).

Table 1 shows population PK simulated median plasma concentrations for the tosylate salt of doses of up to 240 mg given BID (twice daily) or QD (once daily) 6-methyl-5-(1-methyl-1H-pyrazol-5-yl)-N-{[5-(methylsulfonyl)pyridin-2-yl]methyl}-2-oxo-1-[3-(trifluoromethyl)phenyl]-1,2-dihydropyridine-3-carboxamide, showing the median, lower and upper interquartile ranges (lIQ and uIQ), lower and upper 95% CI (i95%CI) and u95%CI) for QD and BID dosing regimens. These all show that the predicted PK levels of 240 mg BID meet the greater than 300nM threshold (dotted line). The median shows the predicted PK levels of 120 mg BID meet the greater than 300nM threshold (dotted line).

It can be seen that in order to achieve a median threshold Cₘᵢₙ (trough plasma concentration) in a human of at least 300 nM, and allowing for up to 125% variance around the predicted Cₘᵢₙ as by Regulatory guidelines (EU and US regulators included), a minimum BID dose of 120 mg is required.

Preferably, in order to achieve a 95% Cl lower threshold Cₘᵢₙ (trough plasma concentration) in a human of at least 300 nM, and allowing for up to 125% variance around the predicted Cₘᵢₙ as by Regulatory guidelines (EU and US regulators included), a minimum BID dose of 240 mg is preferred.

**Table 1: PopPK Simulation plasma concentrations (Cₘᵢₙ) for the tosylate salt**

| | | Simulated PK concentration | | | | |
|---|---|---|---|---|---|---|
| Regimen | Dose (mg) | IIQ | Median | uIQ | l95%Cl | u95%Cl |
| QD | 90 | 31.842 | 50.145 | 72.7995 | 17.21465 | 120.5265 |
| QD | 120 | 41.5185 | 65.6745 | 98.8935 | 23.61045 | 156.442 |
| QD | 150 | 55.4215 | 85.3845 | 121.595 | 27.3528 | 205.093 |
| QD | 180 | 65.76775 | 94.012 | 145.8775 | 37.5023 | 230.9075 |
| QD | 210 | 73.145 | 106.665 | 161.475 | 39.1444 | 296.615 |
| QD | 240 | 83.544 | 131.075 | 187.1575 | 47.0317 | 317.0865 |
| | | | | | | |
| BID | 90 | 196.1375 | 280.64 | 370.1475 | 107.781 | 530.043 |
| BID | 120 | 268.52 | 361.175 | 485.7125 | 155.359 | 727.246 |
| BID | 150 | 338.5225 | 449.28 | 584.6575 | 206.2275 | 825.7995 |
| BID | 180 | 390.1925 | 540.2 | 726.22 | 235.342 | 1012.285 |
| BID | 210 | 452.005 | 636.295 | 861.595 | 256.8515 | 1256.835 |
| BID | 240 | 504.2475 | 705.125 | 943.6825 | 288.7425 | 1369.025 |

### Example 3: the protection of proteinase associated lung damage by alvelestat (a neutrophil elastase inhibitor)

### Summary

Alpha-1 antitrypsin deficiency (AATD) is a genetic condition which can result in reduced/no release of the protective anti-proteinase alpha-1 antitrypsin (AAT). The lack of this major inhibitor of potentially destructive proteinases, results in a persistent proteinase/anti-proteinase imbalance state. Uninhibited proteinase, in particular elastase, activity causes structural changes to the lung and leads to emphysema and airway obstruction. Using an in vitro model of this imbalance allows for physiological assessment of the effect of elastase inhibitors on neutrophil serine proteinases (NSPs) involved in pathophysiology.

This example describes the effect of adding alvelestat into a stimulated neutrophil-fibrinogen buffer model to assess changes in elastase activity by monitoring the release of elastase specific markers of the process.

A model of the proteinase/anti-proteinase imbalance was developed and validated by describing the effect of the physiological inhibitor AAT on uninhibited NSP activity through elastase footprint activity immunoassays. Alvelestat was then added into the model.

Results obtained by adding alvelestat indicate that the compound is successfully and rapidly able to inhibit elastase activity in vitro and more effectively than the physiological inhibitor AAT.

### Introduction

Alpha-1 antitrypsin deficiency (AATD) is a genetic condition arising from a mutation in the SERPINA1 gene [1] which encodes the glycoprotein alpha-1 antitrypsin (AAT) crucial for inhibition of destructive neutrophil serine proteinases. Mutation of the SERPINA1 gene produces AAT variants that lead to little/no secretion into the blood and tissues, thereby reducing physiological control of potentially damaging proteinases.

In healthy tissues, destructive proteinases are modulated by anti-proteinases (mostly AAT), however in AATD , the lack of AAT means these proteinases retain excessive activity; leading to a major proteinase/anti-proteinase imbalance [2] [3] [4].

This proteinase/anti-proteinase imbalance is believed to be the cause of the pathophysiological irreversible emphysematous changes associated with patients with AATD COPD (chronic obstructive pulmonary disease) [4].

Uninhibited proteinases are free to cleave major extracellular matrix (ECM) proteins, such as elastin, collagen and fibrinogen, damaging lung structure and leading to emphysema [5].

Indeed, clinically, an AAT serum concentration of below 11 µM is regarded as key to a non-physiological interaction resulting in increased emphysematous changes and hence clinical lung disease and is termed the putative "at-risk threshold" [6].

Historically, proteinase damage in COPD has been mostly attributed to neutrophil elastase (NE) activity, as demonstrated by animal studies [7].

Recent development of in vivo proteinase activity footprint immunoassays for NE, based upon detection of specific fibrinogen cleavage products in subject plasma, have supported the theory of the importance of the proteinase/anti-proteinase imbalance in AATD [8].

All deficient AAT phenotypes measured for NE activity demonstrated increased evidence of these footprints compared to healthy smoking volunteers, and demonstrated different profiles between AAT phenotypes [8].

It was hypothesised that the addition of alvelestat into the model would provide data on the effect of the drug *in vitro,* including exposure response to support dose ranging used in clinical trial, and support the products of this model as a biomarker of pharmaceutical and hence clinical efficacy.

The primary purpose of this model is to describe changes in elastase activity related to alvelestat NE inhibitor application.

### Materials and Methods:

### Enzyme kinetics

The initial steps involved assessment of the biological activity of the enzymes and inhibitors to be used in the more physiological experiments.

Step 1 involved assessment of the activity of Porcine Pancreatic Elastase using Lineweaver-Burk reciprocal plot analysis [9].

Step 2 Having confirmed the PPE activity, the inhibitory function of a purified batch of AAT (Athens Research & Technology, US) was determined against this enzyme and used for all subsequent experiments.

Step 3 involved using the pure AAT to assess inhibition of pure NE preps (Athens Research and Technology, US) to derive their active function. The enzyme activity was then used to assess the activity of alvelestat.

Activity of alvelestat was calculated by measuring N-succinyl-Ala-Ala-Ala-p-nitroanilide (SlaaapN) cleavage when the compound was titrated against elastase of known concentration and activity [2]. Using these methods, alvelestat activity against NE was concluded to be 88%. Results were determined computationally using Prism software.

### The Proteinase/anti-proteinase imbalance model

Neutrophils from healthy young volunteers were isolated using a method broadly described by Jepsen and Skottun [10].

Neutrophils were then incubated for 15 minutes at 37°C with 550 nM fibrinogen (Calbiochem, San Diego, CA, US), 10 µM N-formyl-L-methionyl-L-leucyl-phenylalanine (fMLP; Sigma-Aldrich, St. Louis, MO, US), as well as concentrations of active AAT between 0-11 µM (Athens Research and Technology, Athens, GA, US) and increasing concentrations of AZD9668 in Roswell Park Memorial Institute media 1640 (RPMI; Sigma-Aldrich; with L-glutamine and sodium bicarbonate) supplemented with Penicillin-streptomycin (1% v/v; Sigma-Aldrich) - Figure 3.

Supernatant was obtained through centrifugation, and NE activity footprints were measured.

### Specific proteinase activity assays

Inverse fluorescence immunoassay methods described previously by Carter *et al.* and Newby *et al.* [8] were utilised to measured neutrophil elastase and proteinase 3 activity footprints.

### Study subjects

Healthy, young (<35 years old) non-smokers blood and plasma was collected with ethical approval under "Investigations of the ageing immune system" (ERN_12-1184) held by the Institute of Inflammation and Ageing, University of Birmingham.

### Results

### 1. Development of a Novel in vitro Model using Isolated Neutrophils in Fibrinogen containing buffer

To generate an appropriate model for assessment of alvelestat, parameters involved in development of a fibrinogen-buffer neutrophil model were individually explored and optimal conditions selected.

### i. Validation of the Aα-Val³⁶⁰ assay with resting neutrophils

To select optimal fibrinogen concentrations, neutrophils were incubated along an uncleaved fibrinogen titration curve, ranging from 0-8797 nM. 8797 nM being the mean human blood fibrinogen concentration described in the literature. The curve was measured alongside a negative control with no neutrophils (see Figure 4).

### ii. Validation of the Aα-Val360 assay with stimulated neutrophils

To determine maximal stimulation a potent stimulator of neutrophils, Cal, [11] was titrated out from 0-2.4 µM and specific proteinase fibrinogen-cleavage product measured.

The NE activity footprint rose rapidly by 43.8 nM (+/- 17.1) following stimulation of neutrophils with 0.3 µM Cal and then also plateaued (Figure 5).

Flow cytometry confirmed neutrophil degranulation and, also, despite strong stimulation, neutrophil viability remained high. Flow cytometry assessment confirmed that DMSO used to dissolve alvelestat did not stimulate the neutrophils to degranulate.

To assess a more physiological stimulus, neutrophils were then incubated with N-formylmethionine-leucyl-phenylalanine (fMLP).

There was a rapid increase in proteinase activity observed with stimulations from 0-1 µM. At 0.1 µM.

At 0.1 µM, NE activity increased by 11.4 nM, and at 1 µM activity further increased by 7.3 nM. Activity continued to increase to 35.0 at 10 µM fMLP, although the rate of increase was less (Figure 6).

Again, flow cytometry assessment confirmed that fMLP stimulated degranulation, with no affect cell viability and DMSO did not stimulate the neutrophils to degranulate.

### 2. Enzymatic activity

### i. Porcine pancreatic elastase activity

Prior to application of inhibitors, activity of PPE was determined by measuring substrate optical density over one hour, then the rate of reaction velocity and substrate cleavage were calculated. The Michaelis-Menten plot demonstrated a parabolic curve, so the reaction can be described by the usual Michaelis-Menten equation and is not cooperative or allosteric [12]. Change in absorbance (at 410 nm) of PPE incubated with 6 concentrations of substrate SlaaapN (N-succinyl-Ala-Ala-Ala-p-nitroanilide - CAS Number: 52299-14-6) over 15 minutes (450 seconds). A Lineweaver-Burk plot was also produced. Using the axis crosspoints obtained computationally, the values for maximal rate (Vₘₐₓ) and the Michaelis constant (Kₘ) could be calculated. N-Succinyl-Ala-Ala-Ala-p-nitroanilide can be a chromogenic substrate for chymotrypsin-like serine protease, pancreatic elastase and serine endopeptidase. Procedures for measuring elastase products using SlaaapN as the substrate are known.

Using these values and the known published value for catalytic efficiency [13], the functional activity of PPE could be determined and this value was used for subsequent experiments. PPE activity was concluded to be 81.5%.

### ii. Alpha-1 Antitrypsin Activity

Activity of AAT was assessed kinetically by measuring SlaaapN cleavage by PPE of a known activity, following the incubation of a known active molar concentration of PPE with AAT.

Over time the amount of SlaaapN cleaved increased. As AAT:[active PPE] molar ratio increased, where higher concentrations of inhibitor were present, lower rates of cleavage occurred. With no AAT, PPE activity was 100%, and a molar concentration of 1.208 Mol AAT to 1 Mol PPE was required to neutralise PPE activity.

This meant 1.208 moles of AAT were required to inhibit 1 mol of active PPE. If AAT is fully active, PPE should be inhibited at 1:1. It can therefore be calculated that the functional AAT activity is therefore 83%.

### iii. Neutrophil Elastase activity

Activity of NE was then assessed kinetically by measuring SlaaapN cleavage by NE when inhibited with known active molar concentration AAT. Over time the amount of SlaaapN cleaved increased. As [active AAT]:NE molar ratio increased, where higher concentrations of inhibitor were present, lower rates of cleavage occurred.

This meant the pure active AAT inhibited NE at a molar ratio of 0.99:1confirming the NE to be fully active.

### iv. Alvelestat activity

Activity of alvelestat was assessed kinetically by measuring substrate cleavage by elastase of known activity in the presence of the inhibitor, with inhibitor along an inhibition curve. Over time the amount of SlaaapN cleaved decreased.

Kinetic analysis demonstrated that alvelestat is a highly potent and rapid reversible inhibitor of NE [14]. The molar ratio of compound required to fully inhibit NE is 0.87:1, as outlined below.

We can conclude from this that by extrapolation 0.87 mole of alvelestat is required to inhibit 1 mole of NE. Therefore, activity within the errors of the experiment can be determined to be full.

**Table 2. Computational intercept of axis in Figure 7.**

| Best-fit values | |
|---|---|
| Slope | -114.4 |
| X-intercept | 0.8742 |

Legend: Best-fit values of axis intercept and slope obtained by comparing alvelestat:NE ratios against calculated enzymatic activity of NE against SlaaapN in the presence of alvelestat.

3.The Generation of Serine Proteinase Peptides in the presence of Increasing doses of AAT

AAT was added in increasing functional concentrations to the proteinase/anti-proteinase model together with substrate (fibrinogen) and stimulus (fMLP). Pathophysiological concentrations were selected to mimic different disease genotype AAT concentrations as described by the American Thoracic and European Respiratory Societies: 0 µM representing a Pi '*null*' genotype, 2.6-6.5 µM representing a PiZZ genotype, and 10.4-14.3 µM representing a PiSZ genotype [15].

As concentration of AAT increased, the proteinase activity signals reduced for NE and plateaued around 11 µM (Figure 8). Examination of the change in activity by specific proteinase fibrinogen cleavage products (baseline set at 0 µM as 100% active) found that this represented a percentage decrease of 56.7% for NE (Figure 8).

4.The Generation of Serine Proteinase Peptides in the presence of Increasing doses of alvelestat

Alvelestat was added into the model along a dose response curve using pharmacologically active concentrations previously reported in the literature. When reevaluated to take into account enzymatic activity, the effect of active alvelestat concentrations at 1000 nM, 100 nM, 50 nM, 10 nM and 0 nM on the activity of NE activity within a model of proteinase/anti-proteinase imbalance was assessed (n=3).

Data showed that when 10 nM of the compound was added into the model, change in activity defined by specific neutrophil elastase fibrinogen cleavage products (baseline set at 0 µM as 100% active) decreased to 60.1% for NE (Figure 9). When treated with a higher concentration of 1000 nM, NE activity fell further to 27.4% of the untreated value.

### Discussion

Literature describes alvelestat as a potent inhibitor of elastase in vitro [14] [16], and this is supported by data in this report using the proteinase/anti-proteinase biomarker model. It was observed that as the concentration of this anti-elastase increased, the quantity of specific elastase cleaved fibrinogen products rapidly decreased.

However, the inhibition of elastase activity occurred at a lower molar concentration than observed for AAT. This would imply that the compound could inhibit elastase which is not accessible to the larger AAT molecule indicating perhaps a cell membrane bound or intracellular inhibitory component.

There is limited published evidence describing the molecular interactions of alvelestat. We propose that the compound could therefore also be an effective inhibitor of cellular proteinase prior to substrate binding.

### Summary statements

This report demonstrates the effect of alvelestat on proteinase activity within a more physiological model of the proteinase/anti-proteinase imbalance relevant ton alpha-1 antitrypsin deficiency and its treatment.

The proteinase/anti-proteinase model data, collected in this example, supports a role for alvelestat as a potent inhibitor of elastase activity. Moreover the data supports that alvelestat can "normalise" the dysfunction associated with AATD associated disease, being more effective in suppressing elastase that the physiological AAT protein. The data also suggests the mode of action of the inhibitor exceeds that expected suggesting activity against NE is via a different mechanism to physiological inhibitors such as AAT. Alvelestat can, at concentrations achieved by the dosing regimens described in the application, provide NE inhibition greater than AAT at the protein concentration achieved by augmentation treatment in AATD. This supports the use of alvelestat as treatment of AATD, including in patients where augmentation has not worked, either as replacement to augmentation, or in combination with augmentation.

### Further Summary Points:

1. Alvelestat or a salt thereof, at doses of 120-300 mg, and alternatively 220-300 mg, BID, demonstrates activity in inhibiting neutrophil elastase (NE). This can be seen from Figure 1 where about 100% NE inhibition was observed at a plasma concentration of about 300 nM of alvelestat. Simulated PK modelling (Figure 2) suggests that to achieve a plasma concentration of about 300 nM of alvelestat (or a salt thereof) in a subject, a dose of 120-300 mg BID is required. To have greater confidence in achieving a plasma concentration of about 300 nM of alvelestat (or a salt thereof), a dose of 220-300 mg BID is required. Lastly, example 3, shows that Alvelestat or a salt thereof, at doses of 120-300 mg, and alternatively 220-300 mg, BID, demonstrates activity in inhibiting neutrophil elastase (NE), e.g. as measured by A-α-Val³⁶⁰ (Figure 9). Figure 9 shows that alvelestat at a concentration of 300 nm achieves NE inhibition via measuring percentage change in A-α-Val³⁶⁰. Example 3 also shows that A-α-Val³⁶⁰ is a specific product of neutrophil elastase. A-α-Val³⁶⁰ has been shown to be elevated in patients with AATD. A higher concentration of A-α-Val³⁶⁰ is related to an increased severity of disease, e.g. AATD. Thus elevated levels of A-α-Val³⁶⁰ in a patient may identify them as potential responders to treatment with alvelestat.
2. The NE inhibitory efficacy of alvelestat was compared to the NE inhibitory efficacy of AAT, the natural protein. Inhibition of NE by AAT plateaued at 56.7% inhibition (i.e. 43.3% above baseline - Figure 8), whereas inhibition by alvelestat showed greater inhibition at 72.6% (i.e. 27.4% above baseline - Figure 9) at 1000 nM of alvelestat. Here we show via simulated PK Cₘₐₓ concentrations that concentrations of alvelestat at about 1000 nM would be achieved after dosing with the proposed doses / dosing regimens of the present invention (e.g. at doses of 120-300 mg, and 220-300 mg, BID) (Table 3). Table 3 shows selected population PK simulated Cmax plasma concentrations for alvelestat tosylate at doses of alvelestat of 120 mg and 240 mg given BID (twice daily), showing the median, lower and upper interquartile ranges (lIQ and uIQ), lower and upper 95% Cl (l95%Cl) and u95%Cl) for BID dosing regimens. As can be seen, the values in Table 3 are 1000 nM or greater, and thus, the dosing regimens of the present invention provide the concentrations of alvelestat in order to achieve improved suppression of NE for therapeutic purposes compared to AAT at concentrations achieved therapeutically in treatment of alpha-1 deficiency, by use of AAT replacement "augmentation".

**Table 3 PopPK Simulation plasma concentrations (Cₘₐₓ) for the tosylate salt**

| | | Simulated PK concentration (nM) | | | | |
|---|---|---|---|---|---|---|
| Regimen | Dose (mg) | IIQ | Median | uIQ | I95%Cl | u95%Cl |
| BID | 120 | 1323.7 | 1485.25 | 1668.35 | 999.4453 | 2014.123 |
| BID | 240 | 2648.4 | 2953.5 | 3247.175 | 1991.09 | 3937.693 |

3.Alvelestat surprisingly showed higher potency in comparison to the natural inhibitor (AAT). This effect could be due to the ability of alvelestat to inhibit cell surface elastase (which AAT inhibits poorly due to steric hindrance). The effect could also be due to the ability of alvelestat to inhibit exosome containing elastase, which is released on neutrophil activation (but is resistant to AAT), because AAT cannot enter cells so cannot result in intracellular inhibition.

### References:

1. Zorzetto, M., et al., SERPINA1 Gene Variants in Individuals from the General Population with Reduced α1-Antitrypsin Concentrations. Clinical Chemistry, 2008. 54(8): p. 1331.
2. Sinden, N.J. and R.A. Stockley, Proteinase 3 Activity in Sputum from Subjects with Alpha-1 Anti-trypsin Deficiency and COPD. European Respiratory Journal, 2013. 41: p. 1042-1050.
3. Sinden, N.J., et al., Alpha-1-Antitrypsin Variants and the Proteinase/Anti-Proteinase Imbalance in Chronic Obstructive Pulmonary Disease. American Journal of Physiology, Lung Cellular and Molecular Physiology, 2015. 308: p. 12.
4. Stockley, R.A., Neutrophils and Protease/Antiprotease Imbalance. American Journal of Respiratory and Critical Care Medicine, 1999. 160: p. S49-S52.
5. Rao, N.V., et al., Characterisation of Proteinase 3 (PR-3), a Neutrophil Serine Proteinase: Structural and Functional Properties. The Journal of Biological Chemistry, 1991. 266(15): p. 9540-9548.
6. Wewers, M.D., et al., Replacement therapy for alpha 1-antitrypsin deficiency associated with emphysema. N Engl J Med, 1987. 316(17): p. 1055-62.
7. Lucey, E.C., et al., Effect of Combined Human Neutrophil Cathepsin G and Elastase on Induction of Secretory Cell Metaplasia and Emphysema in Hamsters, with in vitro Observations on Elastolysis by these Enzymes. American Review of Respiratory Disease, 1985. 132(2): p. 362-366.
8. Carter, R.I., et al., The Fibrinogen Cleavage Product Aa-Val360, a Specific Marker of Neutrophil Elastase Activity in Vivo. Thorax, 2015. 66: p. 7.
9. Lineweaver, H. and D. Burk, The Determination of Enzyme Dissociation Constants, in The Fertilizer Investigations Unit, Bureau of Chemistry and Soils, United States, Department of Agriculture. 1934. p. 658-66.
10. Jepsen, L.V. and T. Skottun, A Rapid One-step Method for the Isolation of Human Granulocytes from Whole Blood. Scandinavian Journal of Clinical and Laboratory Investigation, 1982. 42(3): p. 235-238.
11. Lefrancais, E., et al., IL-33 is Processed Into Mature Biosactive Forms by Neutrophil Elastase and Cathepsin G. Proceedings of the National Academy of Sciences of the United States of America, 2012. 109(5): p. 6.
12. Michaelis, L. and M.L. Menten, Die Kinetik der Invertinwirkung. Biochemische Zeitschrift, 1913. 26: p. 333-69.
13. Nakajima, K., et al., Mapping the Extended Substrate Binding Site of Cathepsin G and Human Leukocyte Elastase. Studies with Peptide Substrates Related to the Alpha 1-protease Inhibitor Reactive Site. J Biol Chem, 1979. 254(10): p. 4027-32.
14. Stevens, T., et al., AZD9668: Pharmacological Characterization of a Novel Oral Inhibitor of Neutrophil Elastase. Journal of Pharmacology and Experimental Therapeutics, 2011. 339(1): p. 313.
15. American Thoracic Society/European Respiratory Society, American Thoracic Society/European Respiratory Society Statement. American Journal of Respiratory and Critical Care Medicine, 2003. 168(7): p. 818-900.
16. Gunawardena, K.A., H. Gullstrand, and J. Perrett, Pharmacokinetics and safety of AZD9668, an oral neutrophil elastase inhibitor, in healthy volunteers and patients with COPD. Int J Clin Pharmacol Ther, 2013. 51(4): p. 288-304.

## Claims

1. Alvelestat or a salt thereof for use in a method of treating a disease of the respiratory system which is mediated by α-1 antitrypsin deficiency, wherein the disease is selected from chronic obstructive pulmonary disease (COPD), emphysema, asthma and bronchiectasis, in a patient in need thereof who has not responded to previous alpha-1 antitrypsin (AAT) therapy, the method comprising administering an effective amount of alvelestat or a salt thereof to the patient, and wherein the amount of alvelestat or a salt thereof administered is 120-300 mg BID.

2. Alvelestat or a salt thereof for use in a method of treating a disease of the respiratory system which is mediated by α-1 antitrypsin deficiency, wherein the disease is selected from chronic obstructive pulmonary disease (COPD), emphysema, asthma and bronchiectasis, in a patient in need thereof who has not responded to previous alpha-1 antitrypsin (AAT) therapy, the method comprising administering an effective amount of alvelestat or a salt thereof to the patient, and administering alvelestat or a salt thereof in a dosage escalation regime comprising:
- administering alvelestat or a salt thereof at a dose of 60 mg twice daily for a first period of time,
- administering alvelestat or a salt thereof at a dose of 120 mg twice daily thereafter,
wherein the first period is 5-20 days, preferably about one week (7 days).

3. Alvelestat or a salt thereof for use in a method of treating a disease of the respiratory system which is mediated by α-1 antitrypsin deficiency, wherein the disease is selected from chronic obstructive pulmonary disease (COPD), emphysema, asthma and bronchiectasis, in a patient in need thereof who has not responded to previous alpha-1 antitrypsin (AAT) therapy, the method comprising administering an effective amount of alvelestat or a salt thereof to the patient, and administering alvelestat or a salt thereof in a dosage escalation regime comprising:
- administering alvelestat or a salt thereof at a dose of 60mg twice daily for a first period of time,
- administering alvelestat or a salt thereof at a dose of 120 mg twice daily for a second period of time,
- administering alvelestat or a salt thereof at a dose of 180 mg twice daily for a third period of time, and
- administering alvelestat or a salt thereof at a dose of 240 mg twice daily thereafter,
wherein the first, second and third periods are 5-20 days, preferably about one week (7 days).

4. Alvelestat or a salt thereof for use according to claim 1, wherein the amount of alvelestat or a salt thereof administered is 220-300 mg BID.

5. Alvelestat or a salt thereof for use according to claim 1, wherein 120 mg of alvelestat or a salt thereof is administered per administration.

6. Alvelestat or a salt thereof for use according to claim 1, wherein 240 mg of alvelestat or a salt thereof is administered per administration.

7. Alvelestat or a salt thereof for use according to any of claims 1-6, wherein the disease is COPD.

8. Alvelestat or a salt thereof for use according to any of claims 1-6, wherein the disease is emphysema.

9. Alvelestat or a salt thereof for use according to any of claims 1-6, wherein the disease is asthma.

10. Alvelestat or a salt thereof for use according to any of claims 1-6, wherein the disease is bronchiectasis.

11. Alvelestat or a salt thereof for use according to any of claims 1-10, wherein the active ingredient comprises or consists of the tosylate salt of alvelestat.

## Patentansprüche

1. Alvelestat oder ein Salz davon zur Verwendung in einem Verfahren zur Behandlung einer Erkrankung des Atmungssystems, die durch α-1-Antitrypsinmangel vermittelt wird, wobei die Erkrankung ausgewählt ist aus chronisch obstruktiver Lungenerkrankung (COPD), Emphysem, Asthma und Bronchiektasie, bei einem behandlungsbedürftigen Patienten, der auf eine frühere Alpha-1-Antitrypsin (AAT)-Therapie nicht angesprochen hat, wobei das Verfahren das Verabreichen einer wirksamen Menge von Alvelestat oder eines Salzes davon an den Patienten umfasst, und wobei die verabreichte Menge von Alvelestat oder eines Salzes davon zweimal täglich 120-300 mg beträgt.

2. Alvelestat oder ein Salz davon zur Verwendung in einem Verfahren zur Behandlung einer Erkrankung des Atmungssystems, die durch α-1-Antitrypsinmangel vermittelt wird, wobei die Erkrankung ausgewählt ist aus chronisch obstruktiver Lungenerkrankung (COPD), Emphysem, Asthma und Bronchiektasie, bei einem behandlungsbedürftigen Patienten, der auf eine frühere Alpha-1-Antitrypsin (AAT)-Therapie nicht angesprochen hat, wobei das Verfahren das Verabreichen einer wirksamen Menge von Alvelestat oder eines Salzes davon an den Patienten umfasst sowie das Verabreichen von Alvelestat oder eines Salzes davon in einem Dosiserhöhungsschema, umfassend:
- Verabreichen von Alvelestat oder eines Salzes davon in einer Dosis von 60 mg zweimal täglich während eines ersten Zeitraums,
- anschließend Verabreichen von Alvelestat oder eines Salzes davon in einer Dosis von 120 mg zweimal täglich, wobei der erste Zeitraum 5-20 Tage, vorzugsweise etwa eine Woche (7 Tage), beträgt.

3. Alvelestat oder ein Salz davon zur Verwendung in einem Verfahren zur Behandlung einer Erkrankung des Atmungssystems, die durch α-1-Antitrypsinmangel vermittelt wird, wobei die Erkrankung ausgewählt ist aus chronisch obstruktiver Lungenerkrankung (COPD), Emphysem, Asthma und Bronchiektasie, bei einem behandlungsbedürftigen Patienten, der auf eine frühere Alpha-1-Antitrypsin (AAT)-Therapie nicht angesprochen hat, wobei das Verfahren das Verabreichen einer wirksamen Menge von Alvelestat oder eines Salzes davon an den Patienten umfasst sowie das Verabreichen von Alvelestat oder eines Salzes davon in einem Dosiserhöhungsschema, umfassend:
- Verabreichen von Alvelestat oder eines Salzes davon in einer Dosis von 60 mg zweimal täglich über einen ersten Zeitraum,
- Verabreichen von Alvelestat oder eines Salzes davon in einer Dosis von 120 mg zweimal täglich über einen zweiten Zeitraum,
- Verabreichen von Alvelestat oder eines Salzes davon in einer Dosis von 180 mg zweimal täglich über einen dritten Zeitraum, und
- anschließend Verabreichen von Alvelestat oder eines Salzes davon in einer Dosis von 240 mg zweimal täglich, wobei der erste, zweite und dritte Zeitraum 5-20 Tage, vorzugsweise etwa eine Woche (7 Tage) beträgt.

4. Alvelestat oder ein Salz davon zur Verwendung nach Anspruch 1, wobei die verabreichte Menge Alvelestat oder eines Salzes davon zweimal täglich 220-300 mg beträgt.

5. Alvelestat oder ein Salz davon zur Verwendung nach Anspruch 1, wobei 120 mg Alvelestat oder ein Salz davon pro Verabreichung verabreicht werden.

6. Alvelestat oder ein Salz davon zur Verwendung nach Anspruch 1, wobei 240 mg Alvelestat oder ein Salz davon pro Verabreichung verabreicht werden.

7. Alvelestat oder ein Salz davon zur Verwendung nach einem der Ansprüche 1 bis 6, wobei es sich bei der Erkrankung um COPD handelt.

8. Alvelestat oder ein Salz davon zur Verwendung nach einem der Ansprüche 1 bis 6, wobei es sich bei der Erkrankung um ein Emphysem handelt.

9. Alvelestat oder ein Salz davon zur Verwendung nach einem der Ansprüche 1 bis 6, wobei es sich bei der Erkrankung um Asthma handelt.

10. Alvelestat oder ein Salz davon zur Verwendung nach einem der Ansprüche 1 bis 6, wobei es sich bei der Erkrankung um Bronchiektasie handelt.

11. Alvelestat oder ein Salz davon zur Verwendung nach einem der Ansprüche 1 bis 10, wobei der Wirkstoff das Tosylatsalz von Alvelestat umfasst oder daraus besteht.

## Revendications

1. Alvélestat ou un sel de ce dernier pour une utilisation dans une méthode de traitement d'une maladie du système respiratoire qui est médiée par un déficit en α-1 antitrypsine, la maladie étant choisie parmi la bronchopneumopathie chronique obstructive (BPCO), l'emphysème, l'asthme et la bronchiectasie, chez un patient en ayant besoin qui n'a pas répondu à une thérapie précédente à l'alpha-1 antitrypsine (AAT), la méthode comprenant l'administration au patient d'une quantité efficace d'alvélestat ou d'un sel de celui-ci, et la quantité administrée d'alvélestat ou d'un sel de celui-ci étant de 120 à 300 mg BID.

2. Alvélestat ou un sel de celui-ci pour une utilisation dans une méthode de traitement d'une maladie du système respiratoire qui est médiée par un déficit en α-1 antitrypsine, la maladie étant choisie parmi la bronchopneumopathie chronique obstructive (BPCO), l'emphysème, l'asthme et la bronchiectasie, chez un patient en ayant besoin qui n'a pas répondu à une thérapie précédente à l'alpha-1 antitrypsine (AAT), la méthode comprenant l'administration au patient d'une quantité efficace d'alvélestat ou d'un sel de celui-ci, et l'administration d'alvélestat ou d'un sel de celui-ci selon un schéma en escalade de doses comprenant :
- l'administration d'alvélestat ou d'un sel de celui-ci à une dose de 60 mg deux fois par jour pendant une première période de temps,
- l'administration d'alvélestat ou d'un sel de celui-ci à une dose de 120 mg deux fois par jour ensuite,
la première période étant de 5 à 20 jours, de préférence d'environ une semaine (7 jours).

3. Alvélestat ou un sel de celui-ci pour une utilisation dans une méthode de traitement d'une maladie du système respiratoire qui est médiée par un déficit en α-1 antitrypsine, la maladie étant choisie parmi la bronchopneumopathie chronique obstructive (BPCO), l'emphysème, l'asthme et la bronchiectasie, chez un patient en ayant besoin qui n'a pas répondu à une thérapie précédente à l'alpha-1 antitrypsine (AAT), la méthode comprenant l'administration au patient d'une quantité efficace d'alvélestat ou d'un sel de celui-ci, et l'administration d'alvélestat ou d'un sel de celui-ci selon un schéma en escalade de doses comprenant :
- l'administration d'alvélestat ou d'un sel de celui-ci à une dose de 60 mg deux fois par jour pendant une première période de temps,
- l'administration d'alvélestat ou d'un sel de celui-ci à une dose de 120 mg deux fois par jour pendant une deuxième période de temps,
- l'administration d'alvélestat ou d'un sel de celui-ci à une dose de 180 mg deux fois par jour pendant une troisième période de temps, et
- l'administration d'alvélestat ou d'un sel de celui-ci à une dose de 240 mg deux fois par jour ensuite,
la première, la deuxième et la troisième périodes étant de 5 à 20 jours, de préférence d'environ une semaine (7 jours).

4. Alvélestat ou un sel de celui-ci pour une utilisation selon la revendication 1, la quantité administrée d'alvélestat ou d'un sel de celui-ci étant de 220 à 300 mg BID.

5. Alvélestat ou un sel de celui-ci pour une utilisation selon la revendication 1, 120 mg d'alvélestat ou d'un sel de celui-ci étant administrés par administration.

6. Alvélestat ou un sel de celui-ci pour une utilisation selon la revendication 1, 240 mg d'alvélestat ou d'un sel de celui-ci étant administrés par administration.

7. Alvélestat ou un sel de celui-ci pour une utilisation selon l'une quelconque des revendications 1 à 6, la maladie étant la BPCO.

8. Alvélestat ou un sel de celui-ci pour une utilisation selon l'une quelconque des revendications 1 à 6, la maladie étant un emphysème.

9. Alvélestat ou un sel de celui-ci pour une utilisation selon l'une quelconque des revendications 1 à 6, la maladie étant un asthme.

10. Alvélestat ou un sel de celui-ci pour une utilisation selon l'une quelconque des revendications 1 à 6, la maladie étant une bronchiectasie.

11. Alvélestat ou un sel de celui-ci pour une utilisation selon l'une quelconque des revendications 1 à 10, le principe actif comprenant le sel tosylate de l'alvélestat ou en étant constitué.
